(19) Europäisches Patentamt
European Patent Office
Office européen des brevets

(11) EP 0 589 949 B1

(12) EUROPÄISCHE PATENTSCHRIFT

(45) Veröffentlichungstag und Bekanntmachung des Hinweises auf die Patenterteilung:
**21.08.1996 Patentblatt 1996/34**

(21) Anmeldenummer: **92911774.5**

(22) Anmeldetag: **17.06.1992**

(51) Int Cl.⁶: **C07K 11/02**, A61K 38/12

(86) Internationale Anmeldenummer:
**PCT/EP92/01304**

(87) Internationale Veröffentlichungsnummer:
**WO 93/00362 (07.01.1993 Gazette 1993/02)**

# (54) NEUE PEPTIDE (DIDEMNINE), IHRE HERSTELLUNG UND VERWENDUNG

NEW PEPTIDES (DIDEMNINS), THEIR PREPARATION AND THEIR USE

PEPTIDES NOUVEAUX (DIDEMNINES), LEUR PREPARATION ET LEUR UTILISATION

(84) Benannte Vertragsstaaten:
**AT BE CH DE DK FR GB IT LI NL SE**

(30) Priorität: **20.06.1991 DE 4120327**

(43) Veröffentlichungstag der Anmeldung:
**06.04.1994 Patentblatt 1994/14**

(73) Patentinhaber: **BASF Aktiengesellschaft**
**67063 Ludwigshafen (DE)**

(72) Erfinder:
- **EMLING, Franz**
  **D-6700 Ludwigshafen (DE)**
- **HAUPT, Andreas**
  **D-6370 Oberursel (DE)**
- **KLUGE, Michael**
  **D-6701 Kallstadt (DE)**
- **KRONER, Matthias**
  **D-6719 Eisenberg-Steinborn (DE)**
- **HAAS, Gerhard**
  **D-7867 Wehr (DE)**
- **SCHMIDT, Ulrich**
  **D-7000 Stuttgart (DE)**
- **GRIESSER, Helmut**
  **D-7016 Gerlingen (DE)**
- **RIEDL, Bernd**
  **D-7000 Stuttgart 80 (DE)**

(56) Entgegenhaltungen:
- **JOURNAL OF MEDICINAL CHEMISTRY, vol. 34, no. 2, February 1991, Washington, DC (US); JOUIN et al., pp. 486-491**
- **SYNTHESIS, April 1991, Stuttgart (DE); SCHMIDT et al., pp. 294-300**
- **ANNUAL REPORTS IN MEDICINAL CHEMISTRY, vol. 25, 1990, Washington, DC (US); CAUFIELD et al., pp. 195-204**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 589 949 B1

## Beschreibung

Die vorliegende Erfindung betrifft neue Peptide, deren Herstellung sowie deren Verwendung bei der Bekämpfung von Krankheiten.

Aus Tunicaten der Art Trididemnum Solidum und Trididemnum Cyanophorum sind verschiedene Verbindungen isoliert und in ihrer Struktur aufgeklärt worden (US 4 493 796, US 4 548 814, US 4 782 135, EP 48 149, EP 393 883, Annual Reports in Medicinal Chemistry 25, 195 (1990)), die antivirale und antineoplastische Wirkung besitzen. Einige dieser Verbindungen wurden auch synthetisch hergestellt wie die Didemmine A, B und C (Synthesis 1991, 294) und Nordidemnin und synthetische Analoga (J. Med. Chem. 34, 486 (1991)).

Es wurde nun gefunden, daß Peptide der Formel I

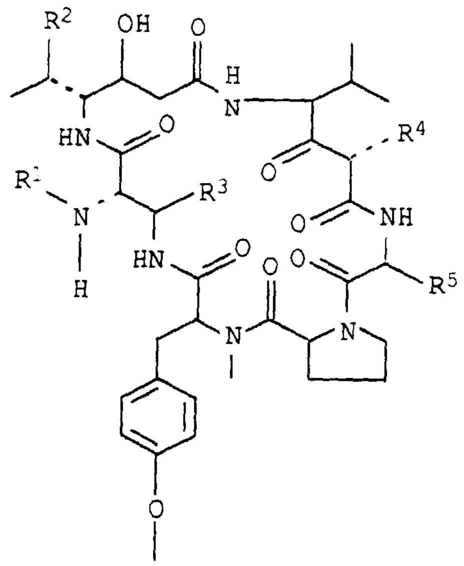

I,

worin

- $R^1$ eine Aminoschutzgruppe, einen linearen, verzweigt-kettigen oder alicyclischen gesättigten oder ungesättigten aliphatischen, aliphatisch-aromatischen oder aromatischen Acylrest mit 1 bis 30 C-Atomen, der durch Fluor-, Nitro-, Oxo-, Hydroxy-, $C_1$-$C_4$-Alkoxy- oder eine gegebenenfalls geschützte Aminogruppe substituiert sein kann,
- $R^2$ ein Wasserstoffatom, eine Methyl- oder Ethylgruppe,
- $R^3$ ein Wasserstoffatom oder eine Methylgruppe,
- $R^4$ ein Wasserstoffatom oder eine Methylgruppe und
- $R^5$ ein Wasserstoffatom oder eine $C_1$-$C_4$-Alkylgruppe

bedeuten, sowie deren Salze mit physiologisch verträglichen Säuren einfacher herstellbar sind und eine verbesserte Wirkung besitzen.

Der N,O-Dimethyltyrosylrest kann in dem Molekül die R- oder S-Konfiguration besitzen.

Von den Verbindungen der Formel I sind diejenigen bevorzugt, in denen

- $R^1$ H, aliphatisches $C_1$-$C_{10}$-Acyl, eine urethanische Aminoschutzgruppe, ein gegebenenfalls durch Cl oder OH substituierter Benzoylrest, eine freie oder N-termial geschützte D-Aminoacylgruppe oder der Sarcosylrest,
- $R^2$ $CH_3$, $C_2H_5$,
- $R^3$ ein Wasserstoffatom oder eine Methylgruppe,
- $R^4$ $CH_3$ und
- $R^5$ $CH_2$-$CH(CH_3)_2$ und $CH(CH_3)_2$

bedeuten.

Als physiologisch verträgliche Säuren sind insbesondere zu nennen: Salzsäure, Zitronensäure, Weinsäure, Milchsäure, Phosphorsäure, Methansulfonsäure, Essigsäuren Ameisensäure, Maleinsäure, Fumarsäure, Äpfelsäure, Bernsteinsäure, Malonsäure, Schwefelsäure, L-Glutaminsäure, L-Asparaginsäure, Brenztraubensäure, Schleimsäure, Benzoesäure, Glucuronsäure, Oxalsäure, Ascorbinsäure, Acetylglycin.

Die neuen Verbindungen lassen sich nach bekannten Methoden herstellen.

So kann man die Verbindungen sequentiell aus Aminosäurederivaten oder durch Verknüpfung geeigneter kleiner

Fragmente aufbauen. Beim sequentiellen Aufbau wird die Peptidkette bzw. Peptolidkette stufenweise um jeweils ein Aminosäurederivat verlängert. Bei der Fragmentkupplung können Fragmente unterschiedlicher Länge miteinander verknüpft werden, wobei die Fragmente ihrerseits wiederum durch sequentiellen Aufbau aus Aminosäurederivaten oder durch Fragmentkupplung gewonnen werden können. Die cyclischen Verbindungen werden nach Synthese der offenkettigen Peptide bzw. Peptolide durch eine Cyclisierungsreaktion erhalten.

Sowohl beim sequentiellen Aufbau, als auch bei der Fragmentkupplung müssen die Bausteine durch Bildung einer Amidbindung bzw. einer Esterbindung verknüpft werden. Hierzu eignen sich enzymatische und chemische Methoden.

Chemische Methoden zur Amidbindungsbildung sind ausführlich behandelt bei Müller, Methoden der Organischen Chemie Vol. xv/2, pp 1 - 364, Thieme Verlag, Stuttgart, 1974; Stewart, Young, Solid Phase Peptide Synthesis, pp 31 - 34, 71 - 82, Pierce Chemical Company, Rockford, 1984; Bodanszky, Klausner, Ondetti, Peptide Synthesis, pp 85 - 128, John Wiley & Sons, New York, 1976, und anderen Standardwerken der Peptidchemie. Besonders bevorzugt sind die Azidmethode, die symmetrische und gemischte Anhydridmethode, in situ erzeugte oder präformierte Aktivester (z. B. Cyan-thiopyridylester) und die Amidbindungsbildung mit Hilfe von Kupplungsreagenzien (Aktivatoren), insbesondere Dicyclohexylcarbodiimid (DCC), Diisopropylcarbodiimid (DIC), 1-Ethoxycarbonyl-2-ethoxy-1,2-dihydrochinolin (EEDQ), 1-Ethyl-3-(3-dimethylaminopropyl)-carbodiimidhydrochlorid (EDCI), n-Propanphosphonsäureanhydrid (PPA), N,N-Bis(2-oxo-3-oxozolidinyl)amidophosphorsäurechlorid (BOP-Cl), Diphenylphosphorylazid (DPPA), Castro's Reagenz (BOP), O-Benzotriazolyl-N,N,N',N'-tetramethyluronium-Salze (HBTU), 2,5-Diphenyl-2,3-dihydro-3-oxo-4-hydroxythiophendioxid (Steglichs Reagenz ; HOTDO) und 1,1'-Carbonyl-diimidazol (CDI). Die Kupplungsreagenzien können allein oder in Kombination mit Additiven wie N,N'-Dimethyl-4-aminopyridin (DMAP), N-Hydroxybenzotriazol (HOBt), N-Hydroxybenzotriazin (HOOBt), N-Hydroxysuccinimid (HOSu) oder 2-Hydroxypyridin eingesetzt werden.

Chemische Methoden zur Esterbindungsbildung sind ausführlich behandelt bei Müller, Methoden der Organischen Chemie Vol. E5, pp 656-773, Thieme Verlag Stuttgart, 1985). Besonders bevorzugt ist die Umsetzung von Carbonsäure und Alkohol mit Dicyclohexylcarbodiimid (DCC) und N,N-Dimethyl-4-aminopyridin (DMAP).

Während bei der enzymatischen Peptidsynthese normalerweise auf Schutzgruppen verzichtet werden kann, ist für die chemische Synthese ein reversibler Schutz der an der Bildung der Amid- bzw. Esterbindung nicht beteiligten reaktiven funktionellen Gruppen der beiden Reaktionspartner erforderlich. Bei den chemischen Peptidsynthesen werden drei literaturbekannte Schutzgruppentechniken für Amine bevorzugt: Die Benzyloxycarbonyl(Z)-, die t-Butyloxycarbonyl(Boc)- und die 9-Fluorenylmethyloxycarbonyl(Fmoc)-Schutzgruppentechnik. Für OH-Gruppen eignen sich besonders die t-Butyl-, die Benzyl-, die 2-Chloracetyl- und die t-Butyldimethyl-sityl-Schutzgruppen.

Die Seitenkettenschutzgruppen der trifunktionellen Aminosäurederivate bzw. Peptid- oder Peptolidbausteine werden so gewählt, daß sie nicht notwendigerweise zusammen abgespalten werden. Eine ausführliche übersicht über Aminosäureschutzgruppen gibt Müller, Methoden der Organischen Chemie, Vol. XV/1, pp 20-906, ThiemeVerlag, Stuttgart, 1974.

Die neuen Verbindungen besitzen eine sehr gute anti-virale und anti-neoplastische Wirkung und lassen sich daher gegen neoplastische Erkrankungen und Autoimmunerkrankungen sowie zur Bekämpfung und Prophylaxe von Infektionen und Abstoßungsreaktionen bei Transplantationen einsetzen.

Die Wirkung der neuen Verbindungen ist sehr selektiv.

Die erfindungsgemäßen Verbindungen können in üblicher Weise oral oder parenteral (subkutan, intravenös, intramuskulär, intraperitoneal) verabfolgt werden.

Die Dosierung hängt vom Alter, Zustand und Gewicht des Patienten sowie von der Applikationsart ab. In der Regel beträgt die tägliche Wirkstoffdosis zwischen etwa 10 und 1000 mg/kg Körpergewicht bei oraler Gabe und zwischen etwa 0,1 und 35 $mg/m^2$ Körperoberfläche bei parenteraler Gabe.

Die neuen verbindungen können in den gebräuchlichen galenischen Applikationsformen fest oder flüssig angewendet werden, z.B. als Tabletten, Filmtabletten, Kapseln, Pulver, Granulate, Dragees, Suppositorien oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 1 bis 90 Gew.-%.

Die folgenden Beispiele sollen die Erfindung näher erläutern:

Beispiel 1

Herstellung der Verbindung I ($R^1$= Benzyloxycarbonyl, $R^2$=$C_2H_5$, $R^3$=$CH_3$, $R^4$=$CH_3$, $R^5$=$CH_2$-$CH(CH_3)_2$, $X^1$=O, $X^2$=O, S-Konfiguration)

o-Monochloracetyl-L-hydroxyisovaleriansäurechlorid (1)

11,8 g (0,1 mol) L-Hydroxyisovaleriansäure wurden in 50 ml $CH_2Cl_2$ bei 0°C vorgelegt; dann wurden 11,3 g (0,1 mol) Chloracetylchlorid in 50 ml $CH_2Cl_2$, dann 8 ml (7,9 g, 0,1 mol) Pyridin zugegeben. Man ließ mindestens 20 h bei RT stehen. Nach Verdünnen mit EE wurde mit 1 N $KHSO_4$ und Wasser gewaschen, getrocknet ($MgSO_4$) und im Vakuum eingeengt. Man erhielt 19,8 g (0,1 mol) gelbliches Öl, das für weitere Umsetzungen genügend rein war.

Die so erhaltene chloracetylgeschützte Hydroxyisovaleriansäure (1 Äqv) wurde mit 2 Äqv. Thionylchlorid versetzt und 3 h auf 50°C erwärmt, bis die Gasentwicklung aufhörte. überschüssiges Reagens wurde im Vakuum entfernt. Das Säurechlorid (1) wurde destilliert. Ausbeute 79 %, Sdp. 103°C/10 Torr, $[\alpha]^2\beta$ = -9,4 (c = 2,7, $CH_2Cl_2$).

O-Monochloracetyl-(2RS,4S)-hydroxyisovalerylpropionsäure (2)

56 mmol (11,9) (1) wurden in 80 ml PE gelöst und zu einer Methylmalonesterenolatlösung bei -60°C getropft, die aus 49,3 ml Methylmalonsäurebis(trimethylsilyl)ester in 220 ml THF und 120 ml 1,6 N BuLi (gegen den Indikator Benzylidenbenzylimin) zuvor bei -60°C hergestellt wurde. über Nacht ließ man auf RT kommen, hydrolysierte mit 100 ml 2 N $KHSO_4$ bis zur sauren Reaktion und extrahierte 4 mal mit Ether, alle organischen Phasen wurden getrocknet ($MgSO_4$) und im Vakuum eingeengt. Der verbleibende Sirup wurde in 100 ml Toluol aufgenommen, 2 h gerührt und von unlöslicher Methylmalonsäure abfiltriert, die mit 50 ml Toluol nachgewaschen wurde. Der Toluolextrakt wurde mit gesättigter $NaHCO_3$-Lösung 3 mal (100, 50, 50 ml) intensiv extrahiert; die vereinigten wäßrigen Phasen 1 mal mit 50 ml Toluol gewaschen, mit 80 m $CHCl_3$ unterschichtet und mit festem $KHSO_4$ vorsichtig (Aufschäumen!) angesäuert. Die wäßrig-saure Phase wurde noch 2 mal mit je 80 ml $CHCl_3$ extrahiert, die organische Phase getrocknet ($MgSO_4$) und im Vakuum eingeengt. Man erhielt ein geruchloses, farbloses Öl.
Ausbeute: 11,2 g (44,8 mmol, 80%).
Die β-Ketosäure ist etwa 85 bis 90 % rein und wurde unverzüglich weiterverarbeitet. Eine Lagerung ist auch in der Kälte nicht unbegrenzt möglich.
$^1H$-NMR (80 MHz, $CDCl_3$): δ = 9,3 (s, 1H), 5,35 (d,J = = 4Hz, 0,6H), 5,25 (d, J=4Hz, 0,4H), 4,2 (s, 2H), 3,5-4,0 (m, 1H), 2,0-2,5 (m, 1H), 1,4 (d, J=6Hz, 3H), 1,35 (d, J=7Hz, 3H), 1,0 (m, 6H).

o-Monochloracetyl-(2RS,4S)-hydroxyisovalgrylpropionyl-L-leucintrichlorethylester (3)

12,8 g (51,1 mmol) (2) wurden mit 12,7 g (51,1 mmol) L-Leucintrichlorethylester in 100 ml $CH_2Cl_2$ gelöst, auf 0°C gekühlt und mit 10,5 g (51,1 mmol) festem DCCD versetzt. Nach 2 h bei O°C wurde vom Harnstoff abfiltriert, eingedampft und mit PE/EE 7/3 chromatographiert. Man erhielt 14,0 g (28,3 mmol, 56 %) sauberes Produkt, das nach 2 Tagen fest wurde. Eine Probe konnte aus Diethylether/PE sehr langsam umkristallisiert werden. Fp. 71-95°C (Diastereomerenmischung).

(2RS,4S)-Hydroxyisovalerylpropionyl-L-leucintrichlorethylester (4)

7,00 g (14,1 mmol) (3) wurden in 70 ml Dioxan gelöst und nacheinander mit 2,14 ml (15 mmol) Triethylamin und 2,22 g (15 mmol) Pentamethylenthioharnstoff versetzt und 3 h auf 70°C erwärmt. Danach wurde im Vakuum eingeengt, in EE aufgenommen und wie üblich aufgearbeitet. Das verbleibende, leicht gelbe Öl wurde mit PE/EE 7/3 über eine kurze Kieselgelsäule filtriert, die erhaltene Kristallmasse in 65 ml Ether gelöst, mit 190 ml PE versetzt und im offenen Kolben stehengelassen. Durch sehr langsame, fraktionierende Kristallisation erhielt man farblose Nädelchen vom Schmp. 101-103°C. Ausbeute: 4,6 g (11 mmol, 78 %).

Boc-(3S,4R,5S)-Isostatin (TBDMS)-OH (5)

a) 7,0 g (25,3 mmol) Boc-(3S,4R,5S)-Isostatin-OH (Synthesis (1989), No. 11, 832-835) wurde in 80 ml DMF gelöst. Zu der gekühlten Lösung gab man 11,1 g (163 mol) Imidazol und 10,6 g (70,3 mmol) TBDMS-Cl. Die Mischung wurde 30 min bei RT belassen. Nach Zugabe von 1,2 1 EE wurde die Reaktionsmischung schnell mit IN $KHSO_4$ (200 ml), und viermal mit Wasser (4 x 150 ml) gewaschen, über $MgSO_4$ getrocknet und zur Trockne eingedampft.

Ausbeute: 12,2 g (25,3 mmol), öl
$[\alpha]^{20}_D$= +7,7° (c = 3,7, $CHCl_3$).

b) Das bissilylierte Produkt (12,2 g, 25,3 mmol) wurde in 200 ml Dioxan gelöst und tropfenweise mit 1N NaOH (26 ml) versetzt, bis der pH-Wert der Lösung alkalisch blieb. Dioxan wurde abgedampft, der Rückstand mit 200 ml EE versetzt und bei 0°C unter starkem Rühren 30 ml IN HCl zugegeben. Die wäßrige Phase wurde mit EE (2 x 100

ml) extrahiert und die vereinigten organischen Phasen über $MgSO_4$ getrocknet und zur Trockne eingedampft.

Ausbeute: 9,9 g (25,3 mmol), öl

$[\alpha]^{20}_{D}$= + 1,74 (c = 2,64, $CHCl_3$) N-tert.-Butyloxycarbonyl-O-tert.-butyldimethylsilyl-(3S,4R,5S)isostatyl-(2RS, 4S)-hydroxyisovalerylpropionyl-L-leucintrichlorethylester (6)

580 mg (1,49 mmol) (5) wurden mit 630 mg (1,50 mmol) (4) und 20 mg DMAP in 3 ml $CH_2Cl_2$ gelöst und bei -30°C mit 310 mg (1,49 mmol) DCCD in 2 ml $CH_2Cl_2$ versetzt. Man ließ über Nacht auf RT langsam erwärmen, verdünnte mit Diethylether und filtrierte vom Harnstoff ab. Die organische Lösung wurde schnell wie üblich gewaschen und das verbleibende Öl über eine kurze Kieselgelsäule mit PE/EE 8/2 filtriert.
Ausbeute: 1,11 g (1,40 mmol, 94%) farbloses Öl, $R_f$ (PE/EE 8/2) = 0,6 (Doppelfleck).

| | | | | | |
|---|---|---|---|---|---|
| $C_{35}H_{63}N_2O_9Cl_3Si$ (790,33) | Ber. | C 53,19 | H 8,03 | N 3,54 | Cl 13,46 |
| | Gef. | C 53,31 | H 8,11 | N 3,54 | Cl 13,23 |

(3S,4R,5S)-Isostatyl-(2RS,4S)-hydroxyisovalerylpropionyl-L-leucintrichlorethylester-hydrochlorid (7)

2,0 g (2,53 mmol) (6) wurden in 10 ml Acetonitril gelöst und mit 2 ml wäßriger Flußsäure (ca. 50 %ig) versetzt. Man rührte 4 h bei RT, neutralisierte mit festem Kalilumhydrogencarbonat und extrahierte 3 mal mit Chloroform, trocknete die organischen Extrakte ($MgSO_4$) und dampfte im Vakuum schnell ein. Der Rückstand wurde unverzüglich bei 0°C in 20 ml HCl/Dioxan aufgenommen und 1 h bei RT gerührt. Man engte ein, dampfte 3 mal mit Methylenchlorid ab und trocknete im Hochvakuum. Ausbeute: 1,55 g (2,53 mmol, quant.).

N-Benzyloxycarbonyl-L-threoninphenacylester (8)

25,3 g (0,1 mol) Z-Threonin wurden in 200 ml EE suspendiert und mit 14 ml (0,1 mol) Triethylamin versetzt. Unter Rühren trug man 19,0 g (0,1 mol) festes ω-Bromacetophenon in einer Portion ein und erhielt zunächst eine klare Lösung, die bald trüb wurde. Man ließ 2 d stehen, verdünnte mit 500 ml EE und arbeitete wie üblich auf. Der kristalline Rückstand wurde in 420 ml Isopropanol in der Wärme gelöst, mit 300 ml warmem Wasser versetzt und und 2 d im Dunkeln stehengelassen. Die ausgefallenen weißen, langen Nadeln wurden abfiltriert, nachgewaschen und an der Luft im Dunkeln getrocknet.

Ausbeute: 27,8 g (0,075 mol, 75 %), Fp 129-130°C,
$[\alpha]^{25}_{D}$= -32,2 (c = 2,04, EE)

N-Boc-L-N,O-Dimethyltyrosin (9)

6,0 g NaH-Suspension wurden 3 mal mit PE gewaschen und abdekantiert, mit 50 ml THF übergossen und auf 0°C gekühlt. Dann tropfte man 32 mmol Boc-Tyrosin, gelöst in 110 ml THF, langsam zu und versetzte anschließend mit 16 ml Methyliodid. Die Suspension wurde 25 h unter Lichtausschluß bei RT gerührt, mit 50 ml EE versetzt und mit Wasser vorsichtig hydrolysiert. Man engt im Vakuum auf ein kleines Volumen ein, überschichtete mit EE und säuerte mit 2 N $KHSO_4$ an. Nach Extraktion mit EE werden die organischen Phasen mit 10 %iger Natriumthiosulfatlösung gewaschen, getrocknet ($MgSO_4$) und im Vakuum eingedampft. Ausbeute: 95 %. $^1$H-NMR (80 MHz, $CDCl_3$): δ = 8,5 (s, 1H), 7,2 (d, 2H), 6,9 (d, 2H), 4,7 (m, 1H), 3,8 (s, 3H), 3,2 (m, 2H), 2,75 (s, 3H), 1,4 (s, 9H)

O-(tert.-Butyloxycarbonyl-L-N,O-dimethyltyrosyl)-N-benzyloxycarbonyl-Lthreonin-phenacylester (10)

2,00 g (5,38 mmol) (8), 1,67 g (5,4 mmol) (9) und 70 mg (0,54 mmol) DMAP wurden in 20 ml $CH_2Cl_2$ gelöst, auf -30°C gekühlt und mit 1,13 g (5,5 mmol) DCCD versetzt. Man ließ über Nacht auf RT kommen, filtrierte den Harnstoff ab, engte das Filtrat ein, nahm den Rückstand in Ether auf, ließ 1 h stehen und filtrierte erneut vom Harnstoff ab. Durch eine Kieselgelfiltration mit PE/EE 7/3 erhielt man das genügend reine Peptolid (10).

Ausbeute: 3,5 g (5,28 mmol, 98 %),
$[\alpha]^{25}_{D}$= -28,7 (c = 0,96, $CHCl_3$)

O-(L-N,O-Dimethyltyrosyl)-N-benzyloxycarbonyl-L-threoninphenacylester (11)

3,5 g (5,28 mmol) (10) wurden mit 20 ml 5 N HCl/Dioxan bei 0°C gelöst und nach 10 min weitere 2 h bei RT stehengelassen. Das Dioxan wurde im Vakuum entfernt, der Rückstand in wenig EE gelöst, zu 200 ml Diethylether unter starkem Rühren langsam zugetropft und noch 2 h weitergerührt. Der Niederschlag wurde abgesaugt und im Vakuum getrocknet.
Ausbeute: 2,80 g (4,67 mmol, 88%).

| | | | | |
|---|---|---|---|---|
| $C_{31}H_{35}N_2O_8Cl$ (599.08) | Ber. | C 62,15 | H 5,89 | N 4,67 |
| | Gef. | C 61,56 | H 5,99 | N 4,59 |

Das so erhaltene Produkt wurde in 30 ml Chloroform gelöst und mit 20 ml 1 N $KHCO_3$ geschüttelt. Die wäßrige Phase wurde noch 2 mal mit Chloroform extrahiert, die vereinigten organischen Phasen getrocknet ($MgSO_4$) und im Vakuum eingedampft.
Ausbeute: 2,45 g (4,35 mmol, 93 %) farbloser Schaum, $R_f$ (PE/EE 1/1) = 0,2.

O-(tert.-Butyloxycarbonyl-L-prolyl-L-N,O-dimethyltyrosyl)-N-benzyloxycarbonyl-L-threoninphenacylester (12)

2,35 g (4,18 mmol (11) und 0,94 g (4,38 mmol) Boc-Prolin werden in 6 ml Methylenchlorid gelöst und bei -20°C mit 1,19 g (1,6 ml, 9,2 mmol) N-Ethyldiisopropylamin und 1,17 g (4,6 mmol) BOP-Cl versetzt und langsam über Nacht auf RT erwärmt. Nach Verdünnen mit Methylenchlorid wurde wie üblich aufgearbeitet und mit PE/EE 4/6 über eine kurze Kieselgelsäule filtriert.
Ausbeute: 3,02 g (3,97 mmol, 95 %), $R_f$ (PE/EE 1/1) = 0,4

| | | | | |
|---|---|---|---|---|
| $C_{41}H_{47}N_3O_{11}$ (757.85) | Ber. | C 64,81 | H 6,50 | N 5,53 |
| | Gef. | C 64,71 | H 6,53 | N 5,46 |

O-(tert.-Butyloxycarbonyl-L-prolyl-L-N,O-dimethyltyrosyl)-N-benzyloxycarbonyl-L-threonin (13)

2,80 g (3,68 mmol) (12) wurden in 20 ml 90 %iger wäßriger Essigsäure mit 1,8 g Zinkpulver 4 h bei RT gerührt; vom ungelösten Zink wird abfiltriert und im Vakuum eingeengt. Der Rückstand wurde zwischen EE und 1 N $KHSO_4$ verteilt. Die kombinierten organischen Phasen wurden getrocknet ($MgSO_4$) und im Vakuum eingeengt. Das verbleibende Harz wurde 3 mal mit Toluol im Vakuum abgedampft. Dann wurde in 10 ml 1 N $KHCO_3$ aufgenommen, 2 mal mit Ether gewaschen und die Etherphasen 2 mal mit $H_2O$ zurückgewaschen. Die vereinigten wäßrigen Phasen wurden mit EE überschichtet und unter Kühlung mit 1 N $KHSO_4$ angesäuert. Es wurde noch 2 mal mit EE ausgezogen und die vereinigten organischen Lösungen getrocknet ($MgSO_4$) und im Vakuum eingedampft. Ausbeute: 2,25 g (3,5 mmol, 95 %).

O-(tert.-Butyloxycarbonyl-L-prolyl-L-N,O-dimethyltyrosyl)-N-benzyloxycarbonyl-L-threonyl-(3S,4R,5S)-isostatyl-(2RS,4S)-hydroxyisovalerylpropionyl-L-leucintrichlorethylester (14)

1,55 g (2,53 mmol) (7) und 1,79 g (2,78 mmol) (13) wurden in 10 ml $CH_2Cl_2$ gelöst, auf 0°C gekühlt, 708 mg (2,78 mmol) BOP-Cl und 1,15 g (1,55 ml, 8,9 mmol) N-Ethyl-diisopropylamin zugegeben und über Nacht langsam auf RT erwärmt. Nach Verdünnen mit Methylenchlorid wurde wie üblich aufgearbeitet. Durch Kieselgelfiltration mit PE/EE 1/1 erhielt man 2,43 g (2,02 mmol, 80 %) Hexapeptolid (14).
MS (Fd, 50°C): 1198 (M+1)

| | | | | | |
|---|---|---|---|---|---|
| $C_{57}H_{82}N_5O_{16}$ (1199,66) | Ber. | C 57,06 | H 6,89 | N 5,84 | Cl 8,86 |
| | Gef. | C 57,30 | H 6,82 | N 5,65 | Cl 8,72 |

O-(tert.-Butyloxycarbonyl-L-prolyl-L-N,O-dimethyltyrosyl)-N-benzyloxycarbonyl-L-threonyl-(3S,4R,5S)-isostatyl-(2RS,4S)-hydroxyisovalerylpropionyl-L-leucin (15)

690 mg (0,57 mmol) Trichlorethylester (14) wurden in 20 ml 90 %iger wäßriger Essigsäure gelöst und mit 2 g Zinkpulver über Nacht gerührt. Man filtrierte ab und engte das Filtrat im Vakuum ein. Der Rückstand wurde in EE aufgenommen und mit 1 N $KHSO_4$ und Wasser gewaschen. Die organischen Phasen wurden getrocknet ($MgsO_4$) und

im Vakuum eingedampft. Der verbleibende Rückstand wurde 3 mal mit Toluol im Vakuum abgedampft. Ausbeute: 610 mg (0,57 mmol) Harz.

Cyclo-[N-benzyloxycarbonyl-O-[[N-[(2S,3S,4S)-4-[(3S,4R,5S)-4-amino-3-(hydroxy)-5-methylheptanoyl]oxy-3-(hydroxy)-2,5-dimethylhexanoyl ]-L-leucyl]-L-prolyl-N,O-dimethyl-L-tyrosyl]-L-threonyl] (17)

a) Herstellung des Pentafluorphenylesters (16)

106 mg (0,1 mmol) Hexapeptolidsäure (15) und 22,1 mg (0,12 mmol) Pentafluorphenol wurden in 0,5 ml $CH_2Cl_2$ bei -20°C mit 22,6 mg (0,11 mmol) DCCD in 0,1 ml $CH_2Cl_2$ versetzt. Man ließ über Nacht auf RT kommen. Für die Abspaltung der BOC-Schutzgruppe wurde diese Lösung ohne weitere Behandlung verwendet.

b) Ringschluß

0,1 mmol Pentafluorphenylester (16) wurden auf 1 ml mit Methylenchlorid verdünnt. Bei 0°C wurden dann 0,5 ml Trifluoressigsäure zugespritzt und 3 h bei 0°C gehalten. Lösungsmittel und überschüssige Säure wurden im Vakuum abgezogen und der verbleibende Rückstand in 200 ml Chloroform gelöst.

Diese Lösung wurde in einem Schütteltrichter vorgelegt und mit 50 ml gesättigter Natriumhydrogencarbonat-Lösung 5 min kräftig geschüttelt. Man ließ noch 1 h stehen, trennte die Chloroformschicht ab und wusch die wäßrige Phase noch 3 mal mit je 30 ml Chloroform nach. Die organischen Lösungen wurden getrocknet ($MgSO_4$) und im Vakuum eingeengt. Durch Chromatographie mit PE/EE 35/65 wurden die epimeren Ringe getrennt.

Ausbeute: 48 mg (0,051 mmol, 51 %) (25)-Hip-Epimeres; 23 mg (0,024 mmol, 24 %) (2R)-Hip-Epimeres.
(2S)-Hip-Epimeres $[\alpha]_D^{25}$ = -147,5 (c = 0,72, EE)
MS: 950 (M+1, 24%)

| | | | | |
|---|---|---|---|---|
| $C_{50}H_{71}N_5O_{13}$ (950.14) | Ber. | C 63,21 | H 7,53 | N 7,37 |
| | Gef. | C 63,13 | H 7,55 | N 7,32 |

Beispiel 2

Cyclo-[N-hexanoyl-O-[[N-[(2S,3S,4S)-4-[(3S,4R,5S)-4-amino-3-(hydroxy)-5-methylheptanoyl]oxy-3-(hydroxy)-2,5-dimethyl-hexanoyl]-L-leucyl]-L-prolyl-N,O-dimethyl-L-tyrosyl]-L-threonyl] (18)

550 mg (0,158 mmol) (17) wurden in 40 ml 90 %iger wäßriger Essigsäure gelöst, mit 1,1 ml 1 N HCl versetzt und mit 150 mg Pd/C unter Normaldruck 6 h hydriert. Danach wurde wie üblich aufgearbeitet und der Rückstand 2 mal mit 5 ml Toluol im Vakuum abgedampft. Das Hydrochlorid blieb als weißes Pulver zurück. Nach der Hydrierung erhielt man in jedem Fall das stabilere (2S)-Hip-Epimere.

Eine Lösung von 500 mg des so erhaltenen Hydrochlorids (0,58 mmol) in 10 ml absolutem $CH_2Cl_2$ wurde bei -10°C mit 0,09 ml Hexansäurechlorid (0,65 mmol) und 0,33 ml Collidin (2,5 mmol) versetzt. Der Ansatz wurde 2 h bei -10°C und 1 h bei RT gerührt. Das $CH_2Cl_2$ wurde im Vakuum abgezogen, der Rückstand in EE gelöst und mit 1 N $H_2SO_4$- und N $KHCO_3$-Lösung gewaschen. Die organische Phase wurde mit $MgSO_4$ getrocknet und eingeengt. Umkristallisation des Rückstandes aus EE/PE 1:1 ergab 400 mg reines (18). Das Filtrat wurde eingeengt und durch MPLC (EE/PE 7/3) gereinigt. Gesamtausbeute: 480 mg = 90 %.

Analog Beispiel 1 und 2 lassen sich folgende Verbindungen I herstellen:

A: $X^1$ = O, $X^2$ = O, $R^2$ = $C_2H_5$, $R^3$ = $CH_3$, $R^4$ = $CH_3$, $R^5$ = $CH_2$-$CH(CH_3)_2$ S-Konfiguration

| Nr. | $R^1$ |
|---|---|
| 3 | $CH_3$-CO |
| 4 | H-CO |
| 5 | $CH_3$-$CH_2$-CO |
| 6 | $CH_3$-$(CH_2)_{14}$-CO |
| 7 | Z |
| 8 | BOC |

(fortgesetzt)

| Nr. | $R^1$ |
|---|---|
| 9 | $CH_3$-$CH_2$-O-CO |
| 10 | Z-N-Me-D-Ala- |
| 11 | p-OH-Benzoyl |
| 12 | $(CH_3)_3$C-CO |
| 13 | HO-$(CH_2)_3$-CO |
| 14 | $C_6H_5$-O-CO |
| 15 | $CF_3$-CO |
| 16 | $(CF_3)_2$-CH-O-CO |
| 17 | $CH_3$-$CH_2$-O-CO-$(CF_2)_2$-CO |
| 18 | p-HO-$C_6H_4$-$(CH_2)_2$-CO |
| 19 | $CH_3$-CO-$CH_2$-CO |
| 20 | $CF_3$-$CH_2$-O-CO |
| 21 | Ac-Sarcosyl |

B: $X^1$ = 0, $X^2$ = 0, $R^2 = C_2H_5$, $R^3 = CH_3$, $R^4 = CH_3$, $R^5 = CH_2\text{-}CH(CH_2)_2$ R-Konfiguration

| Nr. | $R^1$ |
|---|---|
| 22 | n-Hexanoyl |
| 23 | Ac-N-Me-D-Leu |

C: $X^1$ = 0, $X^2$ = 0, $R^2 = CH_3$, $R^3 = CH_3$, $R^4 = CH_3$, $R^5 = CH_2\text{-}CH(CH_3)_2$ S-Konfiguration

| Nr. | $R^1$ |
|---|---|
| 24 | $CH_3$-CO |
| 25 | $CH_3(CH_2)_4$CO |
| 26 | Naphthyl-1-sulfonyl |
| 27 | H-CO |
| 28 | $CH_3$-$CH_2$-CO |
| 29 | $CH_3$-$(CH_2)_{14}$-CO |
| 30 | Z |
| 31 | BOC |
| 32 | $CH_3$-$CH_2$-O-CO |
| 33 | Z-N-Me-D-Ala |
| 34 | Ac-Sarcosyl |
| 35 | p-HO-Benzoyl |
| 36 | $(CH_3)_3$C-CO |
| 37 | HO-$(CH_2)_3$-CO |
| 38 | $C_6H_5$-O-CO |
| 39 | $CF_3$-CO |
| 40 | $(CF_3)_2$CH-O-CO |
| 41 | $CH_3$-$CH_2$-O-CO-$(CF_2)_2$-CO |
| 42 | p-HO-$C_6H_4$-$(CH_2)_2$-CO |
| 43 | $CH_3$-CO-$CH_2$-CO |
| 44 | $CF_3$-$CH_2$-O-CO |

D: $X^1$= 0, $X^2$ = O, $R^2 = CH_3$, $R^3 = CH_3$, $R^4 = CH_3$, $R^5 = CH_2\text{-}CH(CH_3)_2$ R-Konfiguration

| Nr. | $R^1$ |
|---|---|
| 45 | $CH_3$-CO |
| 46 | $CH_3$-$(CH_2)_4$-CO |
| 47 | $(CH_3)_2$CH-CO |
| 48: | $X^1$ = 0, $X^2$ = 0, $R^1$ = Z-N-Me-D-Leu, $R^2$ = $C_2H_5$ |
| | $R^3$ = $CH_3$, $R^4$ = H, $R^5$ = $CH_2$-$CH(CH_3)_2$, S-Konfiguration |

Beispiel 49

Herstellung der Verbindung I ($R^1$ = Benzyloxycarbonyl, $R^2$ = $C_2H_5$, $R^3$ = $CH_3$, $R^4$ =$CH_3$, $R^5$ = $CH_2$-$CH(CH_3)_2$, $X^1$ = NH, $X^2$ = O, S-Konfiguration)

α N-Benzyloxycarbonyl-βN-[tert.-butyloxycarbonyl-L-N,O-dimethyltyrosyl]-(2S,3R)-diaminobuttersäuremethylester (19)

6 mmol (1,82 g) HCl·Z-Dab-OMe (Hoppe-Seyler's Z. Physiol. Chem. 354, 689 (1973) wurden zwischen EE und $NaHCO_3$-Lösung verteilt und die wäßrige Phase wurde noch zweimal mit EE extrahiert. Man trocknete die vereinigten organischen Phasen mit Magnseiumsulfat, dampfte ein, löste zusammem mit 6,3 mmol (1,95 g) Boc-N,O-di-methyl-L-tyrosin in 15 ml Dichlormethan und versetzte bei -20°C mit 6,3 mmol (1,30 g) DCCD.

Nachdem über Nacht auf RT erwärmt worden war, verdünnte man mit reichlich Diethylether, filtrierte und dampfte ein. Der Rückstand wurde erneut in Ether aufgenommen, 1 h stehengelassen, filtriert und eingedampft. Kieselgelfiltration und Mitteldruckchromatographie (PE/EE = 1/1) ergaben 3,04 g (91 %) farblosen Schaum.

Fp.: 46-48°C; $[\alpha]^2\beta$ = -2,3 (c = 1,1; $CHCl_3$);
DC (PE/EE = 1/1): $R_f$ = 0,33; HPLC (Hex/EE = 1/1): $t_R$= 3,6 min;

α N-Benzyloxycarbonyl-β-N-[tert.-butyloxycarbonyl-L-prolyl-L-N,O-dimethyltyrosyl]-(2S,3R)-diaminobuttersäuremethylester (20)

4,8 mmol (2,68 g (19) wurden in 5 ml Dichlormethan gelöst, mit 10 ml HCl/Dioxan versetzt und 1 h bei RT gerührt. Danach dampfte man ein, nahm in EE auf und schüttelte mit $NaHCO_3$-Lösung kräftig durch. Die wäßrige Phase wurde noch zweimal mit EE extrahiert. Die vereinigten organischen Phasen wurden mit Magnesiumsulfat getrocknet, filtriert und eingedampft.

Den hierbei erhaltenen farblosen Schaum löste man zusammen mit 5 mmol (1,08 g) Boc-L-prolin in 15 ml Dichlormethan und versetzte bei -20°C zuerst mit 5 mmol (1,27 g) Bop-Cl und dann mit 11 mmol (1,42 g) Ethyldiisopropylamin.

Man ließ über Nacht auf RT erwärmen, arbeitete wie üblich auf und filtrierte über Kieselgel. Nach Mitteldruckchromatogrphie (PE/EE = 4/6) erhielt man 2,77 g (88 %) farblosen Schaum.

$[\alpha]^{2o}_D$ = -55,1 (c = 1,0; $CHCl_3$); DC (PE/EE = 4,6): Rf = 0,3; HPLC
(Hex/EE = 4/6: $t_R$ = 4,8 min;

α N-Benzyloxycarbonyl-β-N-[tert.-butyloxycarbonyl-L-prolyl-L-N,O-dimethyltyrosyl]-(2S,3R)-diaminobuttersäure (21)

Zu 3,5 mmol (2,29 g (23) in 15 ml Dioxan/Wasser (2:1) wurden 4 ml lN-NaOH unter pH-Kontrolle (pH < 10) langsam zugetropft. Nach weitgehendem Umsatz (DC-Kontrolle) wurde das Dioxan im Vakuum entfernt und die wäßrige Phase zweimal mit Diethylether extrahiert. Aus der Etherphase konnte gegebenenfalls unumgesetzter Ester zurückerhalten werden, während die Wasserphase mit EE überschichtet und bei 0°C mit festem Kaliumhydrogensulfat auf pH 1 bis 2 angesäuert wurde. Nach mehrfacher Extraktion mit EE, Trocknung der organischen Phasen mit Magnesiumsulfat und Abdampfen des Lösungsmittels erhielt man die entsprechende Carbonsäure.
Ausbeute: 2,1g (93,7 %) farbloser, amorpher Feststoff;

$^{\alpha}$N-Benzyloxycarbonyl-β-N-[tert-butyloxycarbonyl-L-prolyl-L-N,O-dimethyltyrosyl]-(2S,3R)-diaminobutyryl-(2S,4R, 5S)-isostatyl-(2RS,4S)-oxyisovalerylpropionyl-L-leucintrichlorethylester (22)

1,1 mmol (705 mg) (21) und 1,1 mmol 674 mg (7) wurden in 8 ml Dichlormethan gelöst, bei 0°C mit 1,1 mmol (280 mg) Bop-Cl und 3,4 mmol (440 mg) Ethyldiisopropylamin versetzt und über Nacht auf RT erwärmt. Nach üblicher Aufarbeitung und Mitteldruckchromatographie (PE/EE = 3/7) erhielt man 570 mg (43 %) farblosen Schaum.
DC (PE/EE = 3/7): $R_f$ = 0,32; HPLC (Hex/EE = 3/7): $t_R$ = 3,1 min;

$^{\alpha}$ N-Benzyloxycarbonyl-β-N-[tert-butyloxycarbonyl-L-prolyl-L-N,0-dimethyltyrosyl]-(2S,3R)-diaminobutyryl-(25,4R, 55)-isostatyl-(2RS,4S)-oxyisovalerylpropionyl-L-leucin (23)

0,475 mmol Trichlorethylester (22) wurden in 20 ml 90 %iger Wäßriger Essigsäure gelöst und über Nacht mit 2 g Zinkpulver (frisch mit Salzsäure angeätzt und mit Wasser, Ethanol und Ether gewaschen) kräftig gerührt.

Nach Filtration zog man die Essigsäure im Ölpumpenvakuum ab, nahm den Rückstand in EE auf, wusch mit 10 %iger Zitronensäure, trocknete mit Magnesiumsulfat, filtrierte und engte ein. Zur vollständigen Entfernung der Essigsäure wurde der Rückstand noch zweimal mit Toluol eingedampft und im Hochvakuum getrocknet.
Ausbeute: 453 mg (89,3 %) farbloser Feststoff;

Cyclo-[$N^{\alpha}$-benzyloxycarbonyl-$N^{\beta}$ -[[N-[(2S,3S,4S)-4-[(3S,4R,5S)-k-amino-3-(hydroxy)-5-methylheptanoyl]oxy-3-(hydroxy)-2,5-dimethyl-hexanoyl]-L-leucyl]-L-prolyl-N, 0-dimethyl-L-tyrosyl]-(25, 3R)-diaminobutyryl] (24)

0,374 mmol (400 mg) Hexapeptolidsäure werden nach der allgemeinen Arbeitsvorschrift für den Ringschluß umgesetzt.

a) Darstellung des Pentafluorphenylesters und Abspaltung der Boc-Schutzgruppe

400 mg (23) (0,374 mmol) und 0,56 mmol Pentafluorphenol wurden in 2 ml Dichlormethan bei -20°C mit 0,45 mmol DCCD versetzt und über Nacht auf RT erwärmt. Es wurde filtriert und eingeengt, direkt bei 0°C mit 1 ml Trifluoressigsäure versetzt und 1 bis 4 h gerührt. Die Trifluoressigsäure wurde dann im Hochvakuum entfernt und das erhaltene Harz für den Ringschluß eingesetzt. Beide Reaktionsschritte konnten im DC auf vollständigen Umsatz überprüft werden.

b) Ringschluß

Das bei der Boc-Abspaltung erhaltene Harz (0,37 mmol) wurde in 400 ml trockenem Chloroform aufgenommen und 5 min mit 100 ml gesättigter Natriumhydrogencarbonatlösung kräftig geschüttelt. Man ließ 1 h stehen und extrahierte dann die wäßrige Phase noch zweimal mit Chloroform. Die vereinigten organischen Phasen wurden mit Magnesiumsulfat getrocknet, filtriert und eingedampft.

Zur Abtrennung von Dicyclohexylharnstoff nahm man in abs. Diethylether auf, ließ 1 h stehen und filtrierte über eine wattegefüllte Pasteurpipette.

Pentafluorphenol wurde durch Kieselgelfiltration mit Dichlormethan abgetrennt. Das Produkt blieb auf dem Kieselgel haften und konnte anschließend mit EE eluiert werden. Die endgültige Reinigung erfolgte dann durch Mitteldruckchromatographie.

Nach Mitteldruckchromatographie (PE/EE = 35/65) erhielt man 150 mg (42,2 %) farblosen Feststoff.

DC (PE/EE = 35/65): $R_f$ = 0,34; Fp.; 112°C;
$[\alpha]^{20}_{D}$ = -165.3 (c = 0,9; $CHCl_3$); MS: 948,4 (M), 949,4 (M+H);

Cyclo-[$N^{\alpha}$-[Z-N-Me-D-leucyl]-$N^{\beta}$-[[N-[(2S,3S,4S)-4-[(3S,4R,5S)-4-amino-3-(hydroxy)-5-methylheptanoyl]oxy-3-(hydroxy)-2,5-dimethylhexanoyl]-L-leucyl]-L-prolyl-N,D-dimethyl-L-tyrosyl]-(2S,3R)-di-aminobutyryl] (25)

0,084 mmol (24) wurden in 10 ml 90 %iger wäßriger Essigsäure mit 200 µl 1N-HCl und 30 mg Pd/C bei RT und 1 bar 6 h hydriert (DC-Kontrolle).

Abfiltrieren des Katalysators, Abziehen der Essigsäure im Vakuum und zweimaliges Eindampfen mit Toluol ergibt das Cyclopeptolid-hydrochlorid in quantitativer Ausbeute.

Das Cyclopeptolid-hydrochlorid wurde in 0,5 ml Dichlormethan bei 0°C mit 0,12 mmol Z-N-Me-De-Leu-3-cyano-4,6-dimethyl-2-thiopyridylester und 0,12 mmol Triethylamin versetzt. Man rührte noch bei RT über Nacht und arbeitete wie folgt auf.

Man filtrierte zunächst das gelbe Thiopyridonderivat ab und trug das eingeengte Filtrat auf eine kurze Kieselgelsäule auf. überschüssigen Thiopyridylester entfernte man durch Spülen mit PE/EE = 7/3. Anschließend konnte das Produkt mit PE/EE = 2/8 eluiert werden. Das Thiopyridylester-freie Rohprodukt wurde dann in EE mit 10 %iger Zitronensäure, 1 N NaOH (Entfernung des restlichen Thiopyridons) und ges. Kochsalzlösung gewaschen, mit Magnesiumsulfat getrocknet und eingedampft.

Nach Mitteldruckchromatographie (PE/EE = 3/7) erhielt man 62 mg (68,6 %) farblosen Feststoff.

Fp.: 102-103°C, DC (PE/EE = 3/7): $R_f$ = 0,25;
$[\alpha]^{20}_{D}$ = -113 (c = 0,9; $CHCl_3$);

Analog lassen sich (zum Teil unter Verwendung des entsprechenden Norstatinderivates und/oder des entsprechenden D-Tyrosin-Derivates und/oder von (2RS,4S)-Fluorenylmethyloxycarbonyl-aminoisovalerylpropionsäure und/oder von (2S)-2,3-Diaminopropansäure) folgende Verbindungen der Formel I herstellen:

F: $X^1$ = NH, $X^2$ = 0, $R^2$ = $C_2H_5$, $R^3$ = $CH_3$, $R^4$ = $CH_3$, $R^5$ = $CH_2$-$CH(CH_3)_2$ , S-Konfiguration

| Nr. | $R^1$ |
|---|---|
| 51 | Boc-N-Me-D-Leu |
| 52 | Ac-Sarcosyl |
| 53 | $CH_3CO$ |
| 54 | $CH_3(CH_2)_4CO$ |
| 55 | $CF_3CO$ |
| 56 | PhOCO |
| 57 | p-OH-Benzoyl |
| 58 | Z-N-Me-D-Ala |
| 59 | $CH_3CH_2OCO$- |
| 60 | Ac-Gly |
| 61 | Napthalin-2-sulfonyl |
| 62 | N-Me-D-Val |
| 63 | Perfluorpropionyl |
| 64 | Boc |
| 65 | Ethyloxycarbonyl |

G: $X^1$ = NH, $X^2$ = NH, $R^2$ = $CH_3$, $R^3$ = $CH_3$, $R^4$ = $CH_3$, $R^5$ = $CH_2$-$CH(CH_3)_2$, S-Konfiguration

| Nr. | $R^1$ |
|---|---|
| 66 | $CH_3$-CO |
| 67 | $CH_3$-$(CH_2)_4$-CO |
| 68 | Z-N-Me-D-Leu |
| 69 | Z-Sarcosyl |

H: $X^1$ = NH, $X^2$ = NH, $R^2$ = $CH_3$, $R^3$ = H, $R^4$ = $CH_3$, $R^5$ = $CH_2$-$CH(CH_3)_2$, S-Konfiguration

| Nr. | $R^1$ |
|---|---|
| 70 | $CH_3CO$ |
| 71 | $CH_3(CH_2)_4CO$ |
| 72 | Boc-N-Me-D-Leu |
| 73 | $CF_3CO$ |
| 74 | p-OH-Benzoyl |
| 75 | Ac-Sarcosyl |
| 76 | $C(CF_3)_2OCO$ |
| 77 | PhOCO |

I: $X^1$ = NH, $X^2$ = O, $R^2$ = $CH_3$, $R^3$ = H, $R^4$ = $CH_3$, $R^5$ = $CH_2$-$CH(CH_3)_2$,

| Nr. | $R^1$ |
|---|---|
| 78 | $CH_3CO$ |
| 79 | HCO |
| 80 | $CH_3(CH_2)_4CO$ |
| 81 | $(CH_3)_3CCO$ |
| 82 | $CF_3CO$ |
| 83 | p-OH-Benzoyl |
| 84 | Z |
| 85 | Boc |
| 86 | Z-N-Me-D-Ala |
| 87 | Z-N-Me-D-Leu |
| 88 | Ac-Sarcosyl |
| 89 | PhOCO |
| 90 | $C(CF_3)_2OCO$ |
| 91 | $CH_3CH_2OCO$-N-Me-D-Leu |
| 92 | $CF_3CH_2OCO$ |
| 93 | Naphthalin-1-sulfonyl |
| 94 | Naphthalin-2-sulfonyl |
| 95 | Perfluorheptanoyl |

Zytotoxizitätstest

Die Zytotoxizität wurde in einem weit verbreiteten Standardtestsystem für adhärente Zellinien nach der Methode von Flick and Gifford (Kristallviolett-Assay) [J. Immunol. Meth., 1984; 68, 167-175] getestet. Als Zielzellen wurden die humanen Zellinien CX-I (Kolonkarzinom), MX-I (Mammakarzinom) und LX-I (Lungenkarzinom) verwendet.

Die jeweiligen Zellen wurden in flachbödigen Mikrotiterplatten mit 96 Kavitäten in einer Zelldichte von 2-3 x $10^3$ Zellen pro Kavität ausplattiert und 24 h unter Standardkulturbedingungen (RPMI 1640 mit 10 % fötalem Kälberserum und 1 % nicht-essentiallen Aminosäuren) bei 37°C und 5 % $CO_2$/95 % Luft inkubiert. Danach wurden die Zellen weitere 72 h unter denselben Bedingungen, aber in Gegenwart verschiedener Konzentrationen der Test-Verbindungen inkubiert (Zellkontrollen nur in Medium). Die Quantifizierung der zytotoxischen Aktivität erfolgte nach Entfernung des Kulturmediums und damit der nicht-adhärenten, toten Zellen. Die verbliebenen adhärenten Zellen wurden 20 min mit 50 µl einer Kristallviolett-Färbelösung inkubiert und anschließend die Mikrotiterplatten kräftig mit Wasser gespült um den ungebundenen, wasserlöslichen Farbstoff zu entfernen.

Die verbliebenen wasserunlöslichen Farbkristalle wurden pro Kavität mit 100 µl einer Lösung, bestehend aus 50 % EtOH und 0.1 % Essigsäure, gelöst. Die Absorption jeder Kavität wurde mittels eines Mikrotiterplattenphotometers (Titertec, Multiscan, Flow Lab., Meckenheim, BRD) bestimmt und die halbmaximale Wirkkonzentration der jeweiligen Testsubstanz ermittelt.

In diesem Test zeigten die neuen Verbindungen eine gute zytotoxische Wirkung auf die getesteten menschlichen Tumorzellinien.

Die in den Beispielen verwendeten Abkürzungen haben folgende Bedeutungen:

| | |
|---|---|
| Ac: | Acetyl |
| Äqv: | Äquivalent(e) |
| BOC: | t-Butyloxycarbonyl |
| BOP-Cl: | N,N-Bis(2-oxo-3-oxazolidinyl)amidophosphorsäurechlorid |
| Dab: | (2S,3R)-Diaminobuttersäure |
| DC: | Dünnschichtchromatographie |
| DCCD: | Dicyclohexylcarbodiimid |
| DMAP: | N,N-Dimethyl-4-aminopyridin |
| EE: | Essigsäureethylester |
| Hip: | Hydroxyisovalerylpropionsäure |
| Ist: | Isostatin |
| i.Vak.: | im Vakuum |

konz.: konzentriert
MCA: Monochloracetyl
NMR: Nuclear Magnetic Resonance
PE: Petrolether
Tce: Trichlorethyl
RT: Raumtemperatur
TBDMS: tert.-Butyl-dimethyl-silyl
THF: Tetrahydrofuran
Z: Benzyloxycarbonyl

## Patentansprüche

1. Peptide der Formel I

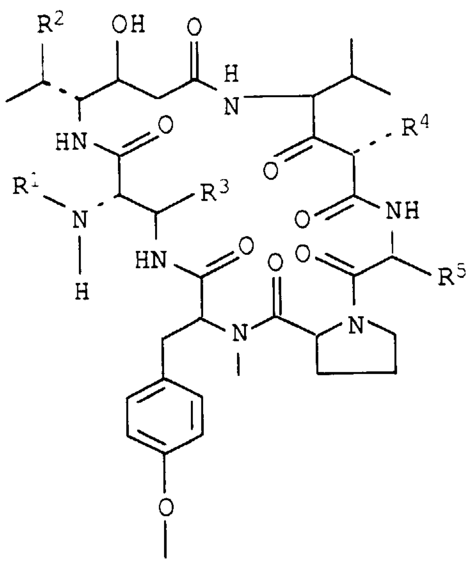

I,

worin

$R^1$ eine Aminoschutzgruppe, einen linearen, verzweigt-kettigen oder alicyclischen gesättigten oder ungesättigten aliphatischen, aliphatisch-aromatischen oder aromatischen Acylrest mit 1 bis 30 C-Atomen, der durch Fluor-, Nitro-, Oxo-, Hydroxy-, $C_1$-$C_4$-Alkoxy- oder eine gegebenenfalls geschützte Aminogruppe substituiert sein kann,
$R^2$ ein Wasserstoffatom, eine Methyl- oder Ethylgruppe,
$R^3$ ein Wasserstoffatom oder eine Methylgruppe,
$R^4$ ein Wasserstoffatom oder eine Methylgruppe und
$R^5$ ein Wasserstoffatom oder eine $C_1$-$C_4$-Alkylgruppe

bedeuten, sowie deren Salze mit physiologisch verträglichen Säuren.

2. Peptide der Formel I gemäß Anspruch 1 zur Verwendung bei der Bekämpfung von Krankheiten.

## Revendications

1. Peptides de formule I

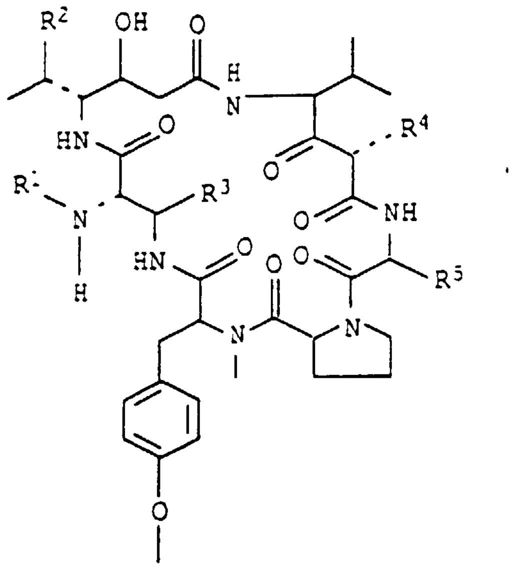

I,

dans laquelle

$R^1$ représente un groupe protecteur du groupe amino, un radical acyle en C1-C30, aliphatique, linéaire, à chaîne ramifiée ou alicylique saturé ou insaturé, un radical aliphatique-aromatique ou aromatique qui peut être substitué par le fluor, des groupes nitro, oxo, hydroxy, alcoxy en C1-C4 ou un groupe amino éventuellement protégé,

$R^2$ représente un atome d'hydrogène, un groupe méthyle ou éthyle,

$R^3$ représente un atome d'hydrogène ou un groupe méthyle,

$R^4$ représente un atome d'hydrogène ou un groupe méthyle et

$R^5$ représente un atome d'hydrogène ou un groupe alkyle en C1-C4,

ainsi que leurs sels d'acides acceptables pour l'usage pharmaceutique.

2. Peptides de formule I selon la revendication 1 pour l'utilisation dans le traitement de maladies.

**Claims**

1. A peptide of the formula I

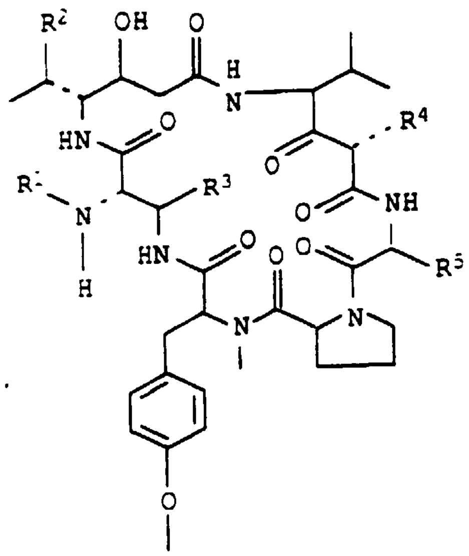

I,

where

$R^1$ is an amino-protective group, a linear, branched or alicyclic saturated or unsaturated aliphatic, aliphatic-aromatic or aromatic acyl radical which has 1-30 carbons and can be substituted by fluorine, nitro, oxo, hydroxyl, $C_1$-$C_4$-alkoxy or unprotected or protected amino,
$R^2$ is hydrogen, methyl or ethyl,
$R^3$ is hydrogen or methyl,
$R^4$ is hydrogen or methyl and
$R^5$ is hydrogen or $C_1$-$C_4$-alkyl,

and the salts thereof with physiologically tolerated acids.

**2.** A peptide of the formula I as claimed in claim 1 for use for controlling diseases.